# EUROPEAN PATENT APPLICATION

(11) **EP 1 424 082 A1**
(43) Date of publication of application: **02.06.2004**
(21) Application number: 02741405.1
(22) Date of filing: 03.07.2002
(51) Int. Cl.: A61K 48/00, A61K 38/18, A61K 45/00, A61K 35/12, A61K 35/32, A61L 27/00, A61P 19/00

(54) **METHOD OF REGENERATING BONE/CHONDRAL TISSUES BY TRANSFERRING TRANSCRIPTIONAL FACTOR GENE**

(30) Priority: 27.07.2001 JP 2001227979
(71) Applicant: National Institute of Advanced Industrial Science and Technology, Tokyo 100-8921 (JP); Japan Science and Technology Agency, Kawaguchi-shi, Saitama 332-0012 (JP)
(72) Inventor: UEMURA, Toshimasa, Tsukuba-shi, Ibaraki 305-0044 (JP); TATEISHI, Tetsuya, Tsukuba-shi, Ibaraki 305-0042 (JP); KOJIMA, Hiroko, Tsukuba-shi, Ibaraki 305-0031 (JP)
(74) Representative: VOSSIUS & PARTNER
(86) International application number: PCT/JP2002/006727
(87) International publication number: WO 2003/011343

(57) **Abstract**

This invention provides a method of rapid and adequate culture of cells isolated from a body to effectively construct bone/cartilage tissues and implants containing the bone/cartilage tissues constructed by the aforementioned method. In this method, osteo-/chondro-inducible transcription factor genes are transfected into bone-marrow-derived cells isolated from a body using an adenoviral or a retroviral vector to grow on adequate scaffolds. The constructed bone/cartilage tissues are transplanted into a body together with the scaffolds. Thus, they can be used as substitutional bone/cartilage implants.

SEQUENCE LISTING

<110> National Institute of Advanced Industrial Science and Technology
      Japan Science and Technology Corporation

<120> Method for regeneration of bone/cartilage tissue by using gene
transmission of transcription factors

<130> PH-1480-PCT

<150> JP2001-227979
<151> 2001-07-27

<160> 2

<170> PatentIn Ver. 2.1

<210> 1
<211> 1557
<212> DNA
<213> Mus musculus

<220>
<221> CDS

<400> 1
ggatccacca tgcgtattcc tgtagatccg agcaccagcc ggcgcttcag ccccccctcc 60
agcagcctgc agcccggcaa gatgagcgac gtgagcccgg tggtggctgc gcagcagcag 120
caacagcagc agcagcaaca gcagcagcaa caacagcaac agcaacaaca gcagcagcag 180
cagcagcagc agcaggaggc ggccgcagca gcagcggcgg cagcggcggc ggcagcagcg 240

gcggcggccg cagtgccccg attgaggccg ccgcacgaca accgcaccat ggtggagatc 300
atcgcggacc acccggccga actggtccgc accgacagtc ccaacttcct gtgctccgtg 360
ctgccctcgc actggcggtg caacaagacc ctgcccgtgg ccttcaaggt tgtagccctc 420
ggagaggtac cagatgggac tgtggttacc gtcatggccg ggaatgatga gaactactcc 480
gccgagctcc gaaatgcctc cgctgttatg aaaaaccaag tagccaggtt caacgatctg 540
agatttgtgg gccggagcgg acgaggcaag agtttcacct tgaccataac agtcttcaca 600
aatcctcccc aagtggccac ttaccacaga gctattaaag tgacagtgga cggtccccgg 660
gaaccaagaa ggcacagaca gaagcttgat gactctaaac ctagtttgtt ctctgatcgc 720
ctcagtgatt tagggcgcat tcctcatccc agtatgagag taggtgtccc gcctcagaac 780
ccacggccct ccctgaactc tgcaccaagt ccttttaatc cacaaggaca gagtcagatt 840
acagatccca ggcaggcaca gtcttcccca ccgtggtcct atgaccagtc ttacccctcc 900
tatctgagcc agatgacatc cccatccatc cactccacca cgccgctgtc ttccacacgg 960
ggcaccgggc tacctgccat cactgacgtg cccaggcgta tttcagatga tgacactgcc 1020
acctctgact tctgcctctg gccttcctct ctcagtaaga agagccaggc aggtgcttca 1080
gaactgggcc ctttttcaga ccccaggcag ttcccaagca tttcatccct cactgagagc 1140
cgcttctcca acccacgaat gcactaccca gccaccttta cctacacccc gccagtcacg 1200
tcaggcatgt ccctcggcat gtccgccacc actcactacc acacgtacct gccaccaccc 1260
taccccggct cttcccaaag ccagagtgga cccttccaga ccagcagcac tccatatctc 1320
tactatggta cttcgtcagc atcctatcag ttcccaatgg tacccggggg agaccggtct 1380
ccttccagga tggtcccacc atgcaccacc acctcgaatg gcagcacgct attaaatcca 1440
aatttgccta accagaatga tggtgttgac gctgacggaa gccacagcag ttccccaact 1500
gttttgaatt ctagcggcag aatggatgag tctgtttggc ggccatattg aggatcc 1557

<210> 2
<211> 1804
<212> DNA
<213> Mus musculus

<220>

<221> CDS

<400> 2
agatctccat gcttcattcg cctcacaaac aaccacagaa ccacaagtgc ggtgcaaact 60
ttctccagga agactgcaag aaggctctgg cgtttaaatg gttaatctct gcaggtcact 120
accagccacc gagaccaacc gagtcattta aggctgcaag cagtatttac aacagagggc 180
acaagttcta tctggaaaaa aaaggaggga ctatggcgtc aaacagcctc ttcagcgcag 240
tgacaccgtg tcagcaaagc ttcttttggg atccgagcac cagccggcgc ttcagccccc 300
cctccagcag cctgcagccc ggcaagatga gcgacgtgag cccggtggtg gctgcgcagc 360
agcagcaaca gcagcagcag cagcagcaac agcagcagca acaacagcaa cagcaacaac 420
agcagcagca gcagcagcag caggaggcgg ccgcagcagc agcggcggca gcggcggcgg 480
cagcagcggc ggcggccgca gtgccccgat tgaggccgcc gcacgacaac cgcaccatgg 540
tggagatcat cgcggaccac ccggccgaac tggtccgcac cgacagtccc aacttcctgt 600
gctccgtgct gccctcgcac tggcggtgca acaagaccct gcccgtggcc ttcaaggttg 660
tagccctcgg agaggtacca gatgggactg tggttaccgt catggccggg aatgatgaga 720
actactccgc cgagctccga aatgcctccg ctgttatgaa aaaccaagta gccaggttca 780
acgatctgag atttgtgggc cggagcggac gaggcaagag tttcaccttg accataacag 840
tcttcacaaa tcctccccaa gtggccactt accacagagc tattaaagtg acagtggacg 900
gtccccggga accaagaagg cacagacaga agcttgatga ctctaaacct agtttgttct 960
ctgatcgcct cagtgattta gggcgcattc ctcatcccag tatgagagta ggtgtcccgc 1020
ctcagaaccc acggccctcc ctgaactctg caccaagtcc ttttaatcca caaggacaga 1080
gtcagattac agatcccagg caggcacagt cttccccacc gtggtcctat gaccagtctt 1140
acccctccta tctgagccag atgacatccc catccatcca ctccaccacg ccgctgtctt 1200
ccacacgggg caccgggcta cctgccatca ctgacgtgcc caggcgtatt tcagatgatg 1260
acactgccac ctctgacttc tgcctctggc cttcctctct cagtaagaag agccaggcag 1320
gtgcttcaga actgggccct ttttcagacc ccaggcagtt cccaagcatt tcatccctca 1380
ctgagagccg cttctccaac ccacgaatgc actacccagc cacctttacc tacacccccc 1440
cagtcacgtc aggcatgtcc ctcggcatgt ccgccaccac tcactaccac acgtacctgc 1500
caccacccta ccccggctct tcccaaagcc agagtggacc cttccagacc agcagcactc 1560
catatctcta ctatggtact tcgtcagcat cctatcagtt cccaatggta cccgggggag 1620

accggtctcc ttccaggatg gtcccaccat gcaccaccac ctcgaatggc agcacgctat 1680
taaatccaaa tttgcctaac cagaatgatg gtgttgacgc tgacggaagc cacagcagtt 1740
ccccaactgt tttgaattct agcggcagaa tggatgagtc tgtttggcgg ccatattgag 1800
atct 1804

## Description

### Technical Field

The present invention relates to a method for regeneration of bone/cartilage tissues by transfection of osteo-/choridro-inducible transcription factor genes. Particularly, the present invention relates to a method of transfection of osteo-/chondro-inducible transcription factor genes to accelerate cell differentiation into the target tissues, thereby effectively constructing bone/cartilage tissues and substitutional bone/cartilage implants produced by the aforementioned method.

### Background Art

Recently, in the field of regenerative medicine, *in vitro* culture and organization of cells from a body have been attempted for reconstructing tissues that are as similar as possible to those in the body and replacing the tissues in the body. In such tissue regeneration, the provision of scaffolds for cell proliferation and acceleration of cell differentiation are indispensable, along with the preservation of cells.

In general, cells exist in a body by adhering to the extracellular matrix, which plays as a scaffold for differentiation and proliferation. Accordingly, suitable scaffolds for cell proliferation and differentiation are necessary in addition to cells, for *in vitro* culture and complete three-dimensional tissue organization. Up to the present, the use of porous biodegradable materials such as porous ceramics as scaffolds has provided sufficient results in the regeneration of hard tissues such as bone and cartilage tissues.

In contrast, a method for rapid differentiation into target tissues *in vitro* is important in the tissue engineering approach for tissue regeneration. Formally, however, cytokines (humoral factors) responsible for cell differentiation have been directly donated to cells or an expression vector carrying cDNA of the target cytokine has been transfected into cells by lipofection method and so on. A certain level of success has been achieved by such methods.

Transfection of cell growth factors, however, did not provide sufficient results by several problems. For example, the target cytokine did not always fully and specifically affect the tissue, and the transfection efficiency was almost 0 in primary cells although gene transfection by lipofection was somewhat successful in established cell lines.

Recently, many researchers have found that transcription factors, particularly transcription factors of runt and Helix-Loop-Helix (HLH) types, play significant roles in osteoblast differentiation. For example, a runt transcription factor, Pebp2alphaA (Pebp2αA)/Cbfa1, and an HLH transcription factor, such as Scleraxis, Id-1, and I-mfa, have been reported to have significant roles in osteoblast differentiation (e.g., Kunikazu Tsuji et al., "Runt transcription factor associated with osteoblast differentiation, Cbfa1/Pebp2αA, and Sox9 function in osteogenesis and chondrogenesis and unusual osteogenesis" Experimental Medicine, 16(11), 25-32, 1998). Special attempts in tissue regeneration utilizing functions of these transcription factors, however, have not yet been reported.

The present inventors have conducted concentrated studies. As a result, they have found that cells can be efficiently induced to differentiate into bone/cartilage tissues by transfecting osteo-/chondro-inducible transcription factors into bone-marrow-derived cells and having them express therein. Further, use of an adenoviral or a retroviral vector for the transfection of a transcription factor results in effective infection to the primary adhesive cells such as bone/cartilage cells, and very effective induction of differentiation at approximately 99% can be achieved. This has led to the completion of the present invention.

The present invention provides the following (1) to (13).
(1) A method for constructing bone/cartilage tissues, wherein an osteo-/chondro-inducible transcription factor gene is transfected into isolated cells, and the isolated cells are allowed to differentiate and proliferate.
(2) The method according to (1) above, wherein the cells are derived from bone marrow.
(3) The method according to (2) above, wherein the bone-marrow-derived cells are mesenchymal stem cells.
(4) The method according to (2) above, wherein the bone-marrow-derived cells are osteoblasts.
(5) The method according to any one of (1) to (4) above, wherein the cells are primary cells.
(6) The method according to (5) above, wherein the cells are isolated from a patient.
(7) The method according to any one of (1) to (6) above, wherein the osteo-/chondro-inducible transcription factor is at least one member selected from the group consisting of Cbfa1, Dlx-5, Bapx1, Msx2, Scleraxis, and Sox9.
(8) The method according to any one of (1) to (7) above, wherein the osteo-/chondro-inducible transcription factor gene is transfected into the cells using an adenoviral or a retroviral vector.
(9) The method according to any one of (1) to (8) above, wherein the cells transfected by the osteo-/chondro-inducible transcription factor genes proliferate in at least one scaffold selected from the group consisting of porous ceramics, collagen, polylactic acid, polyglycolic acid, and a complex thereof.
(10) A method for constructing bone/cartilage tissues comprising the following procedures of:
   1) inducing differentiation of bone-marrow-derived cells isolated from a body with the aid of at least one member selected from the group consisting of dexamethasone, immunosuppressive factors, bone morphogenetic proteins, and osteogenic cytokines;
   2) transfection of the osteo-/chondro-inducible transcription factor gene into the cells using an adenoviral or retroviral vector; and
   3) proliferation of the cells in at least one scaffold selected from the group consisting of porous ceramics, collagen, polylactic acid, polyglycolic acid, and a complex thereof.
(11) Implants containing the bone/cartilage tissues constructed by the method according to any one of (1) to (10) above.
(12) The implants according to (11) above, which further contain at least one scaffold selected from the group consisting of porous ceramics, collagen, polylactic acid, polyglycolic acid, and a complex thereof.
(13) Implants containing cells, in which the osteo-/chondro-inducible transcription factor gene is transfected, and at least one member selected from the group consisting of porous ceramics, collagen, polylactic acid, polyglycolic acid, and a complex thereof.

### Disclosure of the Invention

The present invention is hereafter described in detail.

### 1. Construction of bone/cartilage tissues

The present invention relates to a method of transfection of the osteo-/chondro-inducible (osteo-inducible and/or chondro-inducible) transcription factor gene to isolated cells to construct bone/cartilage (bone and/or cartilage) tissues *in vitro* by inducing differentiation and proliferation of the cells.

### 1.1 Transcription factor

The transcription factors employed in the present invention are the osteo-/chondro-inducible transcription factors that induce immatured cells to differentiate into bone and/or cartilage cells. Examples thereof include Cbfa1, Dlx-5, Bapx1, Msx2, Scleraxis, and Sox9. Cbfa1 is a transcription factor that was cloned by Ogawa et al. at Kyoto University in 1993 and proved to be indispensable for inducing differentiation of the mesenchymal stem cell into the osteoblast by Komori et al. at Osaka University (Komori, T. et al., (1997) Cell 89, 755-764). This Cbfa1 has two isoforms, til-1 and pebp2αA. Dlx-5 is a homolog of the Drosophila distalless (Dll) gene and a transcription factor associated with the ossification of perichondrium and endosporium (Acampora, D. et al., 1999, Development 126, 3795-3809). Bapx1 is a homolog of the Drosophila bagpipe homeobox gene and is associated particularly with differentiation of the mesenchymal stem cell into cartilage cells in the spinal cord and is considered to be a regulatory gene of the Cbfa1 gene (Tribioli, C. et al., 1999, Development 126, 5699-5711). Msx2 is a homolog of the Drosophila muscle segment homeobox (Msh) gene. It is associated with the ossification of calvaria and is considered to be a regulatory gene of the Cbfa1 gene (Satokata, I. et al., 2000, Nature Genet. 24, 391-395). Scleraxis is a transcription factor that is associated with induction of differentiation from the mesenchymal stem cell to cartilage cell or connective tissue (Cserjesi, P. et al., 1995, Development 121, 1099-1110). Sox9 is expressed in cartilage and it regulates expression of the genes associated with cartilage differentiation of, for example, type II collagen (Ng, L. J. et al., 1997, Dev. Biol. 183, 108-121).

In the present invention, the above described osteo-/chondro-inducible transcription factor genes can be prepared by conventional techniques, using their known sequences. For example, cDNA of a target transcription factor can be prepared by extracting RNA from the osteoblast, preparing primers based on a known sequence of the transcription factor gene, and cloning the cDNA by PCR using the RNA and the primers.

### 1.2 Induction of cell differentiation

The cells that are employed in the present invention are immatured cells with differentiation and proliferation ability isolated from a body. Examples thereof include the ES (embryonic stem) cell, the mesenchymal stem cell, and the preosteoblast differentiated from the mesenchymal stem cell. A bone-marrow-derived mesenchymal stem cell is particularly preferable, and the osteoblast is more preferable. Particularly, when the present invention is applied in the field of regenerative medicine, the above-described cell is isolated from the body of a patient. This cell is preferably prepared by removing connective tissues with a conventional technique. Alternatively, cells may grow by primary culture before use.

The cells should be cultured in an appropriate medium for inducing cell differentiation, constructing target tissues. In the case of bone tissue regeneration, for example, at least one member selected from the group consisting of dexamethasone, immunosuppressive factors such as FK-506 and cyclosporine, bone morphogenetic proteins (BMP) such as BMP-2, BMP-4, BMP-5, BMP-6, BMP-7, and BMP-9, and osteogenic humoral factors such as TGFβ is preferably supplemented to the medium to induce cell differentiation into bone cells.

### 1.3 Transfection of transcription factor gene

In the present invention, the osteo-/chondro-inducible transcription factor gene can be transfected into the target cell by a method that is commonly employed for transfection to animal cells. Examples of the methods that can be employed include the calcium phosphate method, lipofection, electroporation, microinjection, and a method using an adenoviral, a retroviral, or a baculoviral vector. From the viewpoints of safety and transfection efficiency, a method using an adenoviral or a retroviral vector is preferable, and a method using an adenoviral vector is most preferable.

The above-mentioned adenoviral or retroviral vector can be constructed according to a known technique. For example, an adenoviral vector can be constructed according to the method of Miyake et al. (Miyake, S. et al., Proc. Natl. Acad. Sci. 93: 1320-1324, 1993). Alternatively, a commercially available kit, such as the Adenovirus Cre/loxP Kit (Takara Shuzo Co., Ltd.), can be used. This kit is used for constructing a recombinant adenoviral vector utilizing a novel regulation system of gene expression (Kanegae Y. et al., 1995, Nucl. Acids Res. 23, 3816) that employs Cre recombinase of P1 phage and its recognition sequence, loxP. This kit can be used to simply construct a recombinant adenoviral vector carrying the transcription factor gene. The multiplicity of infection (moi) of adenovirus infection is 10 or more, preferably 50 to 200, and more preferably around 100 (approximately 80 to 120).

### 1.4 Cell proliferation

The transfected cells should grow in suitable scaffolds in order to construct a complete three-dimensional structure. Examples of materials that can be used for scaffolds include hydroxyapatite or β-TCP (tricalcium phosphate), porous ceramics such as α-TCP, collagen, polylactic acid, polyglycolic acid, and a complex thereof (e.g., a complex of polylactic acid, polyglycolic acid resin, and collagen). These scaffolds may be composed of a simple kind of material or combinations of two or more kinds. Porous ceramics are particularly preferable for scaffolds for tissue regeneration because of their high mechanical strength.

The above-mentioned scaffolds are preferably porous for uniform inoculation of cells. In the present description, the term "porous" refers to a porosity of 40% or higher. The pore size is not particularly limited, although a diameter of 200 µm to 500 µm is preferable for facilitated bone regeneration.

Cells can be proliferated by being inoculated and cultured in the scaffold in accordance with a conventional technique. Cells may be simply inoculated into the scaffold, or inoculated in the form of mixtures with a liquid such as a buffer, physiological saline, a solvent for injection, or a collagen solution. When the cells do not smoothly enter into a pore because of its porous structure of the material, cells may be inoculated under low pressure.

Preferably, the number of cells to be inoculated (inoculation density) is determined in accordance with the types of cells or scaffolds in order to regenerate tissues more efficiently, maintaining the morphology of the cells. For example, the inoculation density is preferably 1,000,000 cells/ml or higher in the case of osteoblasts.

A conventional medium for cell culture, such as MEM medium, α-MEM medium, or DMEM medium, can be suitably selected depending on the type of cell to be cultured and then used for cell culture. FBS (Sigma) and antibiotics such as Antibiotic-Antimycotic (GIBCO BRL) or other substances may be added to the medium. Culture is preferably conducted in the presence of 3% to 10% CO₂ at 30°C to 40°C, and particularly preferably in the presence of 5% CO₂ at 37°C. The culture period is not particularly limited, and it is at least 4 days, preferably at least 7 days, and more preferably at least 2 weeks.

### 2. Implants containing bone/cartilage tissues

The bone/cartilage tissues constructed by the above mentioned method are transplanted or injected into a body together with or separately from the scaffolds. Thus, they can be used as substitutional bone/cartilage implants. Specifically, the present invention provides implants containing bone/cartilage tissues that are constructed *in vitro.*

Bone/cartilage tissues may be transplanted into the body separately from scaffolds, although transplantation with scaffolds is preferable in the present invention. The most suitable scaffold for a given purpose and application site for implants may be selected from the above-mentioned scaffolds. For example, hydroxyapatite is preferable for a transplantation site (or a surgical technique) that requires mechanical strength. In contrast, biodegradable β-TCP or the like is preferable for a transplantation site (or a surgical technique) that does not require mechanical strength.

The configurations and shapes of the implants of the present invention are not particularly limited. Implants can take any desired configurations or shapes, such as sponges, meshes, unwoven fabric products, discs, films, sticks, particles, or pastes. These configurations and shapes may be suitably selected depending on their applications.

The implants of the present invention can suitably contain other components in addition to the bone/cartilage tissues and scaffolds within the scope of the present invention. Examples of such components include: growth factors such as basic fibroblast growth factors (bFGF), platelet-derived growth factors (PDGF), insulin and insulin-like growth factors (IGF), hepatocyte growth factor, (HGF), glial-derived neurotrophic factors (GDNF), neurotrophic factors (NF), hormones, cytokines, bone morphogenetic factors (BMP), transforming growth factors (TGF), and vascular endothelial growth factors (VEGF); bone morphogenetic proteins; inorganic salts such as St, Mg, Ca, and CO₃; organic substances such as citric acid and phospholipid; and drugs.

Bone/cartilage tissues of the implants of the present invention are constructed from the cells to which transcription factor genes have been transfected. Bone/cartilage tissues may be constructed not only prior to the transplantation *(in vitro)* but also after transplantation into bone defects *(in vivo).* The implants of the present invention are highly compatible with bones and of high bone formation ability. Thus, they can be integrated with biological bones and can then restore bone defects immediately after they have been transplanted into a body.

### Brief Description of the Drawings

Fig. 1 is a diagram showing constructions of type I Cbfa1 (pebp2αA) and type II/III Cbfa1 (til-1).
Fig. 2 is a diagram showing a construction of pAxCALNLw cosmid for the Cbfa1 gene transfection.
Fig. 3 is a photograph showing the results of X-gal staining for the evaluation of the LacZ expression level in rat osteoblasts.
Fig. 4 is a graph showing the results obtained by quantifying the expression level of LacZ in rat osteoblasts.
Fig. 5 is a graph showing in the increase of alkaline phosphatase activity in rat osteoblasts after the infection with recombinant adenovirus.
Fig. 6 is a graph showing in the increase of the calcium level in rat osteoblasts after the infection with recombinant adenovirus.
Fig. 7 is an image showing the results of expression level detection regarding Cbfa1 genes by northern hybridization.
Fig. 8 is a graph showing the proliferation of rat osteoblasts after the infection with recombinant adenovirus Fig. 9 is a photograph showing the results of observation of calcification in rat osteoblasts after the infection with recombinant adenovirus by Von Kossa staining.
Fig. 10 is a photograph showing the results of observation of calcification in rat osteoblasts after the infection with recombinant adenovirus by Alizarin Red staining.
Fig. 11 is a photograph showing the results of hematoxylin-eosin staining of a tissue section obtained from the block 4 weeks after transplantation into rat's back subcutaneously.
Fig. 12 is a photograph showing the results of hematoxylin-eosin staining of a tissue section obtained from the block 8 weeks after transplantation into rat's back subcutaneously.
Fig. 13 is a graph showing in the increase of alkaline phosphatase activity in the block after transplantation into rat's back subcutaneously.
Fig. 14 is a graph showing in the increase of the osteocalcin level in the block after transplantation into rat's back subcutaneously.

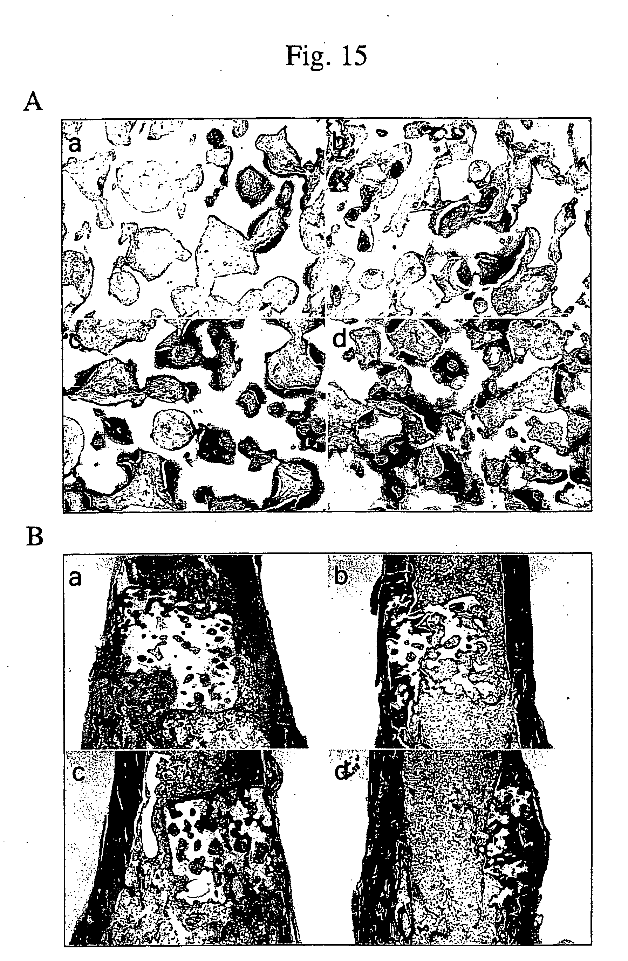

Fig. 15 is a photograph showing the results of hematoxylin-eosin staining of a tissue section obtained from the block after transplantation into rat's back subcutaneously and femur, wherein A-a represents conditions 3 weeks after transplantation of the control into the back; A-b represents conditions 5 weeks after transplantation of the control into the back; A-c represents conditions 3 weeks after transplantation of the Cbfa1 (til-1)-infected cells into the back; A-d represents conditions 5 weeks after transplantation of the Cbfa1 (til-1)-infected cells into the back; B-a represents conditions 3 weeks after transplantation of the control into the femur; B-b represents conditions 8 weeks after transplantation of the control into the femur; B-c represents conditions 3 weeks after transplantation of the Cbfa1 (til-1)-infected cells into the femur; and B-d represents conditions 8 weeks after transplantation of the Cbfa1 (til-1)-infected cells into the femur.
Fig. 16 is a graph showing in the increase of alkaline phosphatase activity in the block after it has been transplanted into rat's back subcutaneously.
Fig. 17 is a graph showing in the increase of the osteocalcin level in the block after transplantation into rat's back subcutaneously.

This description includes a part or all of the contents as disclosed in the description of Japanese Patent Application No. 2001-227979, which is a priority document of the present application.

### Best Modes for Carrying out the Invention

The present invention is hereafter described in more detail with reference to the examples, although the technical scope of the present invention is not limited thereto.

### Example 1: Bone tissue regeneration by transfecting Cbfa1 genes into rat osteoblasts using an adenoviral vector

### 1. Experimental method

### 1) Preparation of adenoviral vector

### (i) Preparation of cDNA of Cbfa1

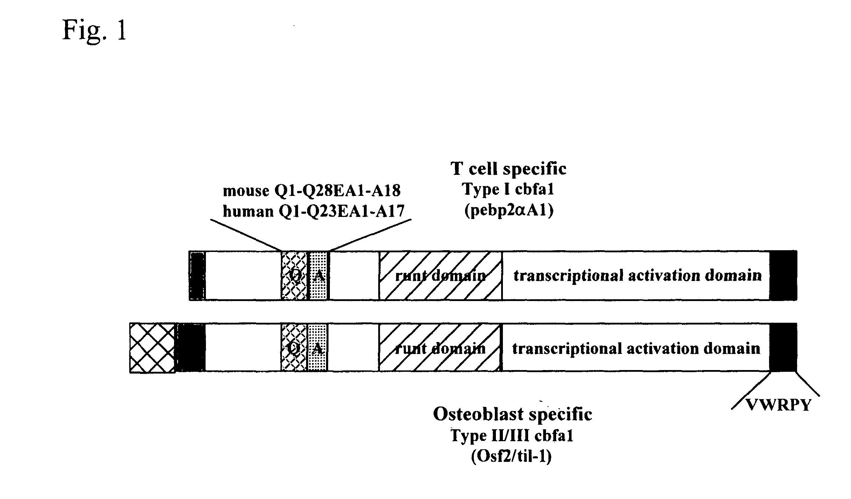

Two types of cDNAs of Cbfa1, i.e., type I: pebp2αA/Cbfa1 (SEQ ID NO: 1) and type II/III: til-1/Cbfa1 (SEQ ID NO: 2), were prepared from two types of plasmids^{*1} (pSG5/IT (mPEBP2aA) and pSG5/KS (mtil-1)) provided by Sumitomo Pharmaceuticals Co., Ltd., ORF of mPEBP2aA was cleaved therefrom using a *Bam*HI restriction enzyme, and ORF of mtil-1 was cleaved therefrom using a *Bgl*II restriction enzyme (Fig. 1).
*1: Two types of Cbfa1 exist, i.e., type I (pebp2αA/Cbfal) expressed specifically in T-cells and type II/III (til-1/Cbfa1) expressed specifically in osteoblasts. Type II/III is longer than type I.

### (ii) Preparation of recombinant adenovirus

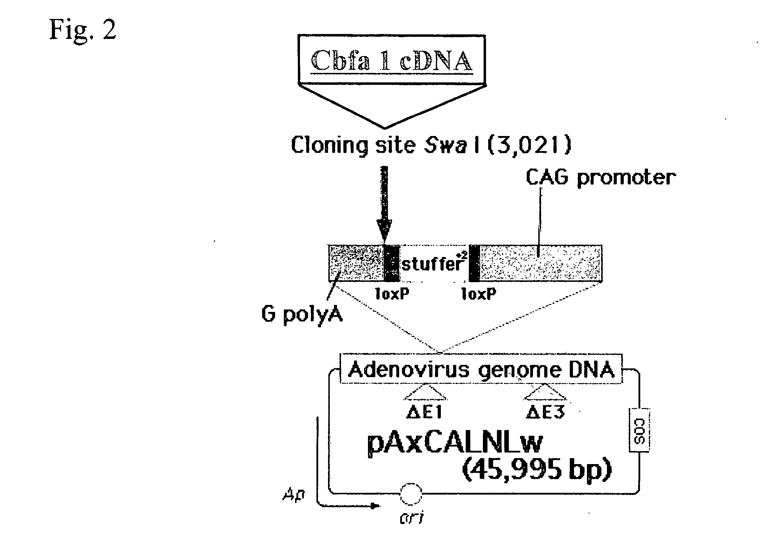

Two types of cDNAs of Cbfa1 were inserted into the SwaI site of the cosmid vector pAxCALNLw using the commercially available Adenovirus Cre/loxP Kit (Takara Shuzo Co., Ltd.), and a recombinant adenoviral vector was prepared according to the manufacturer's instructions attached to the kit (Fig. 2). The titer of the prepared virus was approximately 10¹¹ PFU/ml, and infection efficiency was very high.

### 2) Sampling and culture of bone marrow cells

The rat bone marrow osteoblast (RBMO) was sampled from the back femur of a 6-week-old Fisher rat (male) in accordance with the method of Maniatopoulos et al. (Maniatopoulos, C., Sodek, J., and Melcher, A. H., 1988, Cell Tissue Res. 254, 317-330).

The sampled cells were cultured and in MEM medium (Nacalai Tesque) containing 15% FBS (Sigma) and Antibiotic-Antimycotic (GIBCO BRL) until they became confluent. Subsequently, cells were subcultured into the above mentioned medium containing 5 nM dexamethasone (Sigma), 10 mM β-glycerophosphate (Sigma), and 50 µg/ml ascorbic acid phosphate (Wako) in φ3.5 cm dishes, and they were cultured until the number of cells per dish became approximately 400,000. The subcultured rat osteoblast was infected with the recombinant adenoviral vector prepared as mentioned above at a multiplicity of infection (moi) of 500 on the next day. Uninfected cells or cells infected with the mock vector were prepared as a control.

### 3) Observation of LacZ gene-expressing cell by X-gal staining

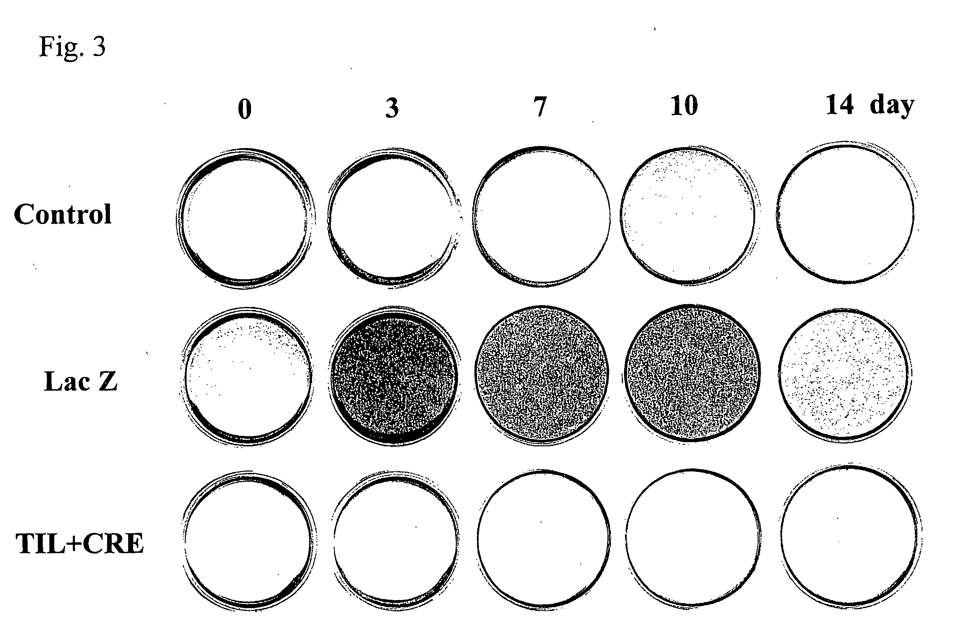

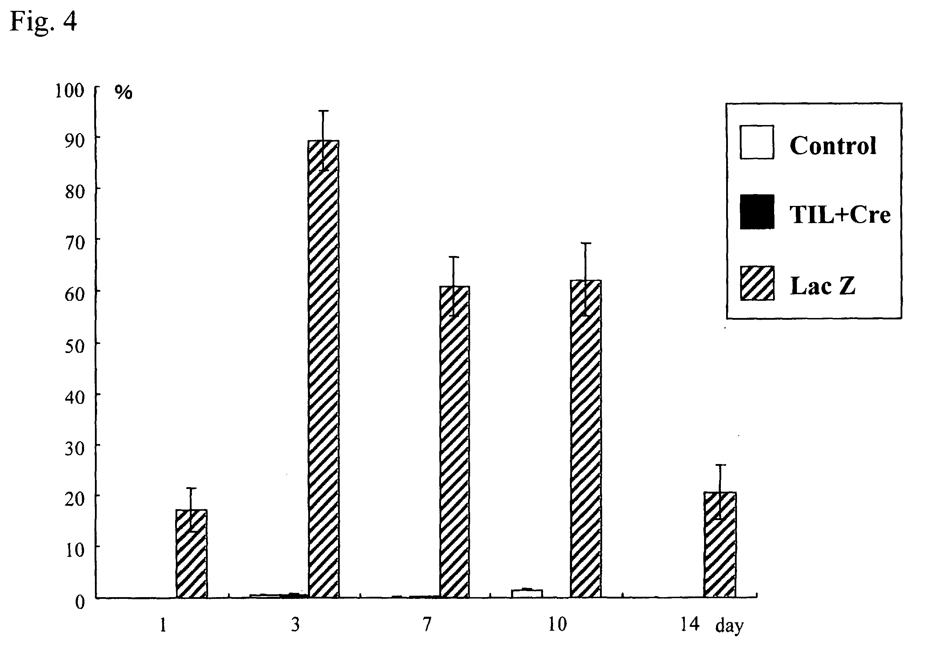

Expression of LacZ in the rat osteoblasts 3 days to 2 weeks after adenovirus infection were observed by X-gal staining in accordance with the method of Scholer et al. (Scholer, H.R. et al., 1989, EMBO J., 8, 2551-2557) (Fig. 3). Further, the stained cells were subjected to image analysis using the NIH image, and the number of expressed cells was determined to evaluate the efficiency of gene transfection (Fig. 4).

### 4) Assay of alkaline phosphatase activity

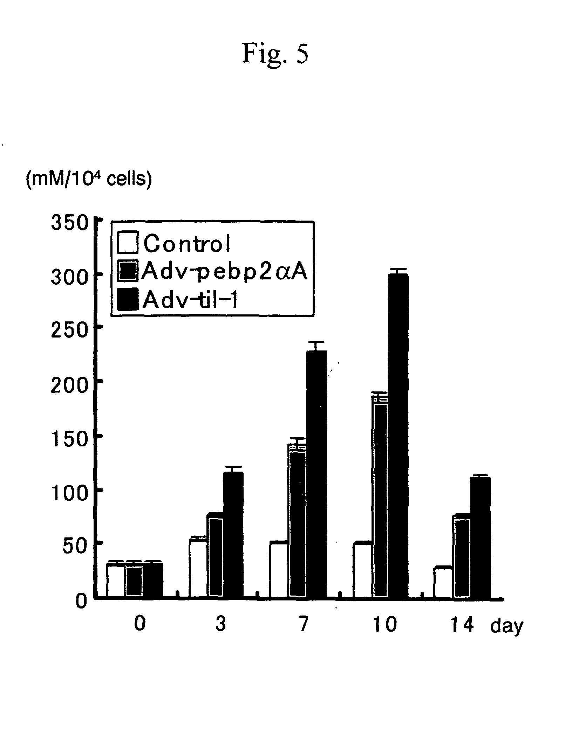

The rat osteoblasts 3 days to 2 weeks after adenovirus infection were washed with 100 mM Tris (pH 7.5) and 5 mM MgCl₂, collected using a scraper, suspended in 500 µl of 100 mM Tris (pH 7.5), 5 mM MgCl₂, and 1% Triton X-100, and then disrupted by sonication. After the sonication, cells were centrifuged at 6,000 g for 5 minutes to recover the supernatant. The supernatant (5 µl in each case) was added to 0.056M 2-amino-2-methyl-1,3-propanediol (pH 9.9), 10 mM p-nitrophenyl phosphate, and 2 mM MgCl₂, the resultants were incubated at 37°C for 30 minutes, and then absorbance at 405 nm was measured using a microplate reader immediately thereafter. The enzyme activity was determined based on the calibration curve prepared using p-nitrophenol from the obtained absorbance (Fig. 5).

### 5) Assay of the calcium level

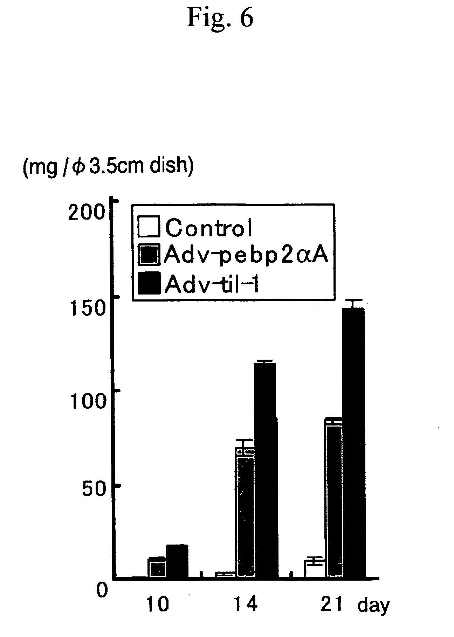

The rat osteoblasts 1 to 3 weeks after adenovirus infection were fixed with 3.7% formalin buffer and then decalcified with 0.6M HCl all day and night. The decalcified solution was diluted, and the calcium level was assayed using a commercially available o-cresol phthalein complexon-containing reagent for calcium analysis (# 587, 360-11, Sigma) according to the manufacturer's instructions (Fig. 6).

### 6) Northern hybridization

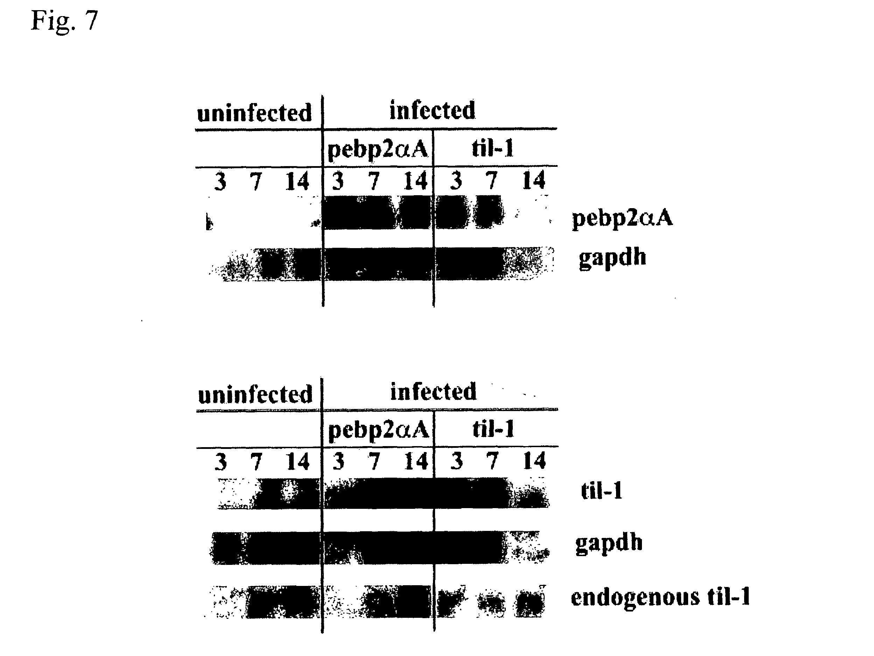

Total RNA was extracted from the rat osteoblasts after adenovirus infection using a commercially available TRIzol reagent (# 15596-10551, GIBCO BRL) according to the manufacturer's instructions. Total RNA (10 µg) was separated on a 1% agarose/5.5% formaldehyde gel and transferred in 20x SSC to the Hybond™-XL membrane (Amersham Pharmacia Biotech). Thereafter, the membrane was heated at 80°C for 2 hours and the irradiated with ultraviolet rays for 2 minutes. The cDNA probes of GAPDH and Cbfa1 were labeled with α-³²PdCTP (3,000 Ci/mmol, Amersham Pharmacia Biotech) using Rediprime™ (Amersham Pharmacia Biotech), and the α-³²PdCTP that had not been incorporated was removed using a MicroSpin™ G-25 Column (Amersham Pharmacia Biotech). This membrane was incubated in the PerfectHyb™ Plus Hybridization Buffer (Sigma) at 68°C for 30 minutes, the labeled cDNA probe (2x10⁶ cpm/ml) was added, and incubation was further carried out at 68°C for 1 hour. The membrane was washed with 2x SSC/0.1% SDS at room temperature for 5 minutes and then washed twice with 0.5x SSC/0.1% SDS at 68°C (20 minutes each). Thereafter, the membrane was exposed to the Kodak XAR film at -80°C overnight (Fig. 7).

### 7) Measurement of the number of cells

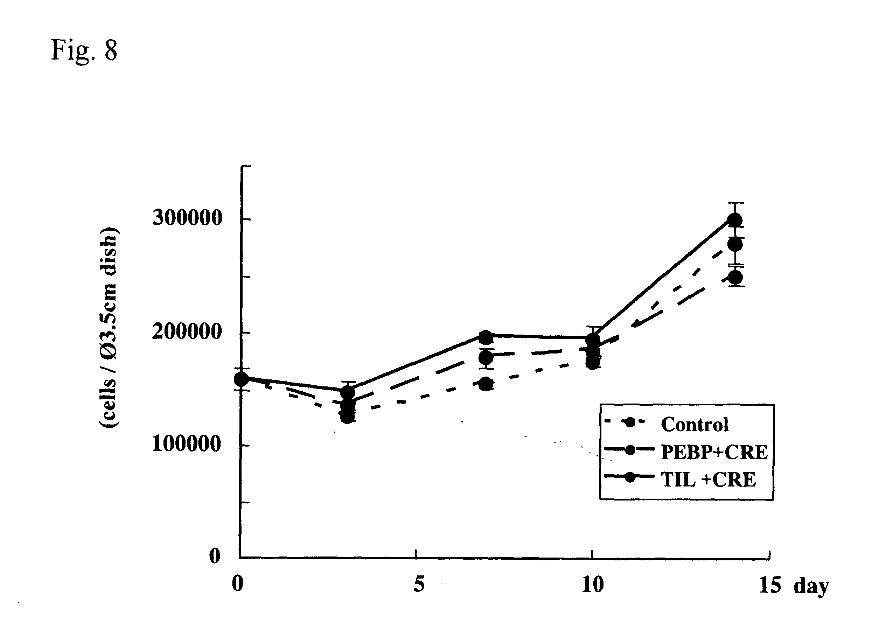

The osteoblasts 3 days to 2 weeks after adenovirus infection were immobilized with PBS containing 1% glutaraldehyde for 5 minutes, washed twice with distilled water, and then stained with 1% crystal violet at room temperature for 30 minutes. After an excess amount of dye was removed by washing three times with distilled water, decolorization was carried out with 10% acetic acid and 1% Triton X-100. The destaining solution was diluted, and absorbance at 595 nm was measured. The number of cells was determined based on the calibration curve ^{*2} from the obtained absorbance (Fig. 8).
*2: The calibration curve was prepared by inoculating cells at an adequate density (duplicate), processing the cells by the above staining technique, and counting the number of tripsinized cells.

### 8) Observation of calcification

The conditions of calcification caused by mineral components secreted from the osteoblasts were observed by Von Kossa staining and Alizarin Red staining.

### (i) Von Kossa staining

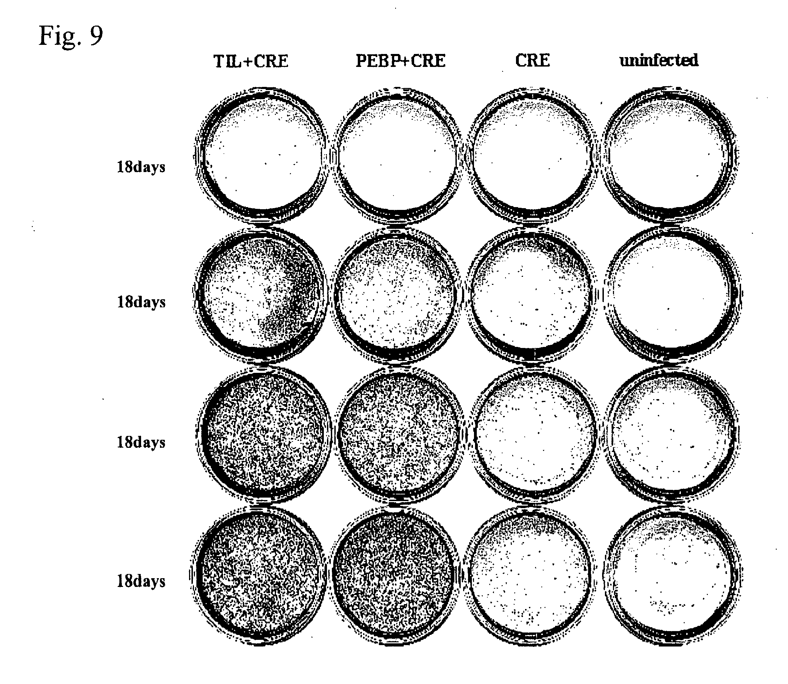

The osteoblasts 1 to 3 weeks after adenovirus infection were fixed with 3.7% formalin buffer for 5 minutes and then rinsed with diluted water. An aqueous solution of 5% silver nitrate was added, and incubation was then carried out for 20 minutes in the dark. Thereafter, incubation was further carried out for 15 minutes in the light, and the resultant was washed thoroughly with distilled water. The results were scanned in a scanner and then compared (Fig. 9).

### (ii) Alizarin Red staining

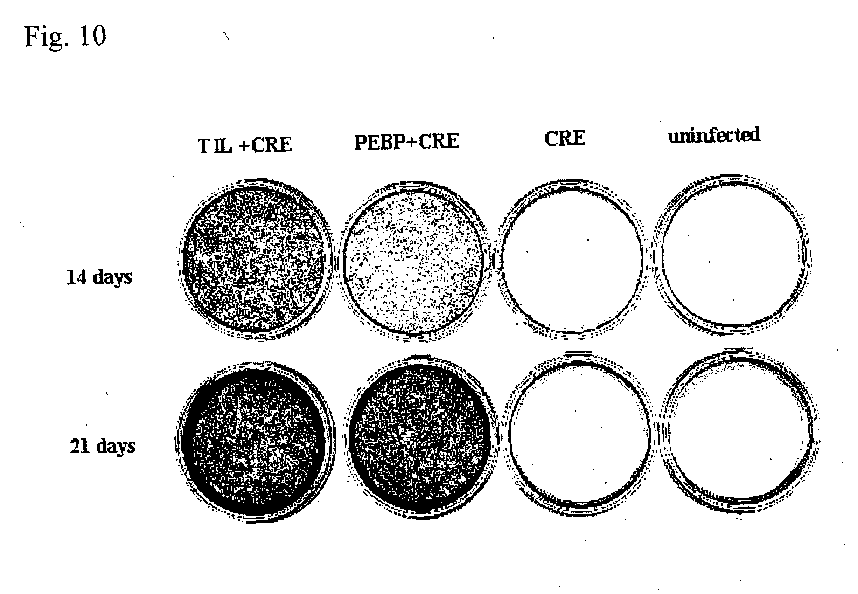

The osteoblasts 1 to 3 weeks after infection were fixed with 3.7% formalin buffer for 5 minutes and then rinsed with diluted water. An aqueous solution of 1% Alizarin Red was added thereto, and incubation was then carried out for 2 minutes. Thereafter, the resultant was washed thoroughly with distilled water. The results were scanned in a scanner and then compared (Fig. 10).

### 2. Results of experimentation

### 1) Expression of lacZ gene

As is apparent from Fig. 3 and Fig. 4, the expression level of the LacZ genes reached its peak 3 days after infection, and the LacZ genes were expressed in approximately 90% of cells. Thereafter, the number of LacZ-expressing cells gradually decreased, although the expression level was maintained in some of the cells (approximately 20%) even 2 weeks after infection.

### 2) Alkaline phosphatase activity

As is apparent from Fig. 5, the alkaline phosphatase activity of the cell in which Cbfa1 (pebp2αA, til-1) had been excessively expressed was higher than that of the control. Particularly, the enzyme activity 10 days after infection became as high as approximately 6 times that of the control.

### 3) Calcium level

As is apparent from Fig. 6, the amount of calcium deposited in the cell in which Cbfa1 (pebp2αA, til-1) overexpressed cell was considerably larger than that in the control.

### 4) Expression of Cbfa1 genes

As is apparent from Fig. 7, a very high level of Cbfa1 gene expression was observed in the cell 3 days after the Cbfa1 (pebp2αA, til-1) recombinant adenovirus infection. Expression of endogenous Cbfa1 genes was observed in the til-1-infected cell.

### 5) Cell proliferation

As is apparent from Fig. 8, the virus titer of the Cbfa1 and Cre recombinant adenovirus infected cell was twice compared to that of the uninfected cell(control). There was no substantial difference in cell proliferation.

### 6) Calcification

As is apparent from the results of Von Kossa staining (Fig. 9) and Alizarin Red staining (Fig. 10), the level of calcification was more advanced in the cell in which Cbfa1 (pebp2αA, til-1) overexpressed cell.

### 3. Conclusion

Accordingly, cDNA of Cbfa1 was very effectively transfected into the osteoblasts with the aid of an adenoviral vector, and the transfected Cbfa1 gene would significantly accelerate the osteoblast differentiation into bone tissues. This culture system was also found to be capable of providing effective ways for *in vitro* tissue regeneration through the use of adequate scaffolds such as porous ceramics.

### Example 2: Experimentation for transplanting the Cbfa1 gene-transfected osteoblasts into a rat's back subcutaneously

### 1. Experimental method

### 1) Transplantation into rat's back subcutaneously

The Cbfa1 gene-transfected rat osteoblasts were cultured using a commercially available β-TCP (tricalcium phosphate) porous block (average pore size: 200 µm in diameter, 5 mm x 5 mm x 5 mm, Olympus) as a scaffold in the following manner, and the composite was transplanted subcutaneously to a rat's back. The confluent rat osteoblasts were treated with trypsin and the cell suspension was then soaked into the β-TCP block under low pressure (100 mHg) at a cell density of 1,000,000/ml. After the cells were cultured for an additional 2 weeks, they were infected with the Cbfa1 (til-1)-recombinant adenoviral vector prepared in Example 1 at an moi of 500, and the infected cells were transplanted subcutaneously to a rat's back on the next day. As the control, blocks comprising cells infected with the mock adenoviral vector were similarly transplanted. The blocks were excised 2, 4, and 8 weeks after transplantation and then subjected to a variety of assays and observations.

### 2) Observation of tissue sections (hematoxylin-eosin staining)

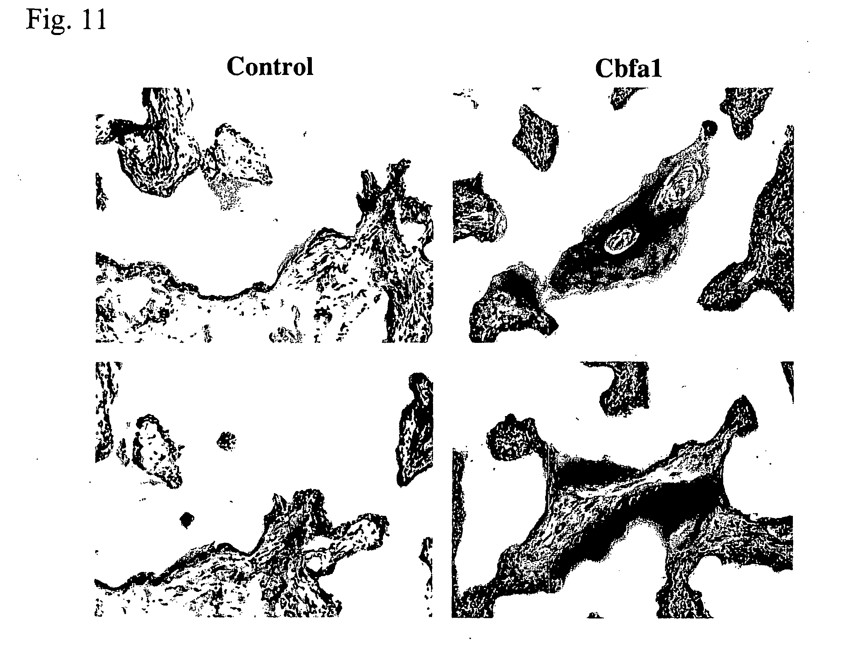

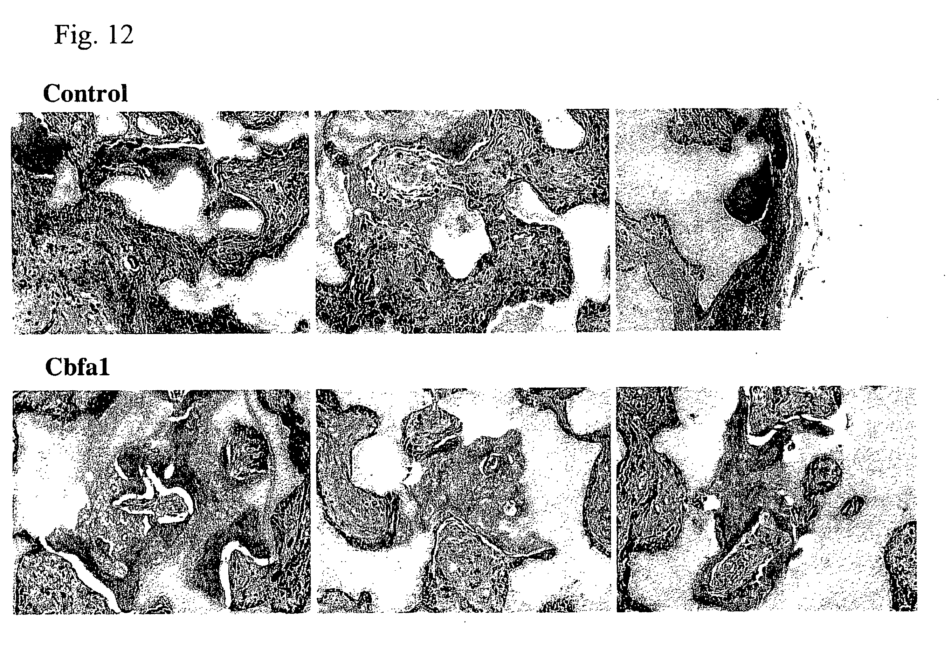

The excised blocks were fixed with 4% paraformaldehyde and 0.05% glutaraldehyde by microwave radiation. On the next day, the resultant was subjected to decalcification in 10% EDTA and 100 mM Tris (pH 7.4), and decalcification was continued for about 1 week. After the decalcification, the blocks were dehydrated in ethanol and then embedded in paraffin. Sctions prepared to a thickness of 5 µm each were deparaffinized and stained with hematoxylin and eosin (Figs. 11 and 12).

### 3) Assay of alkaline phosphatase activity

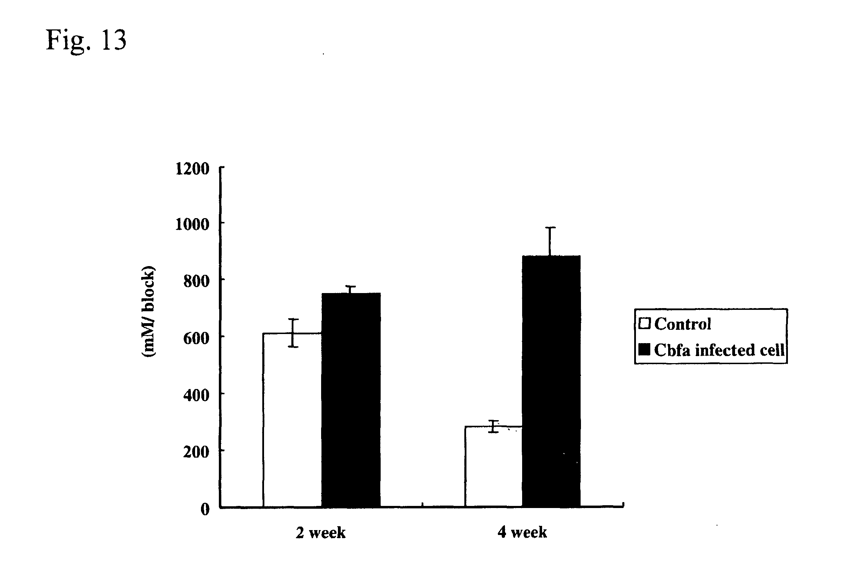

The alkaline phosphatase activities of the excised blocks were assayed in the same manner as in Example 1 (Fig. 13).

### 4) Assay of the osteocalcin level

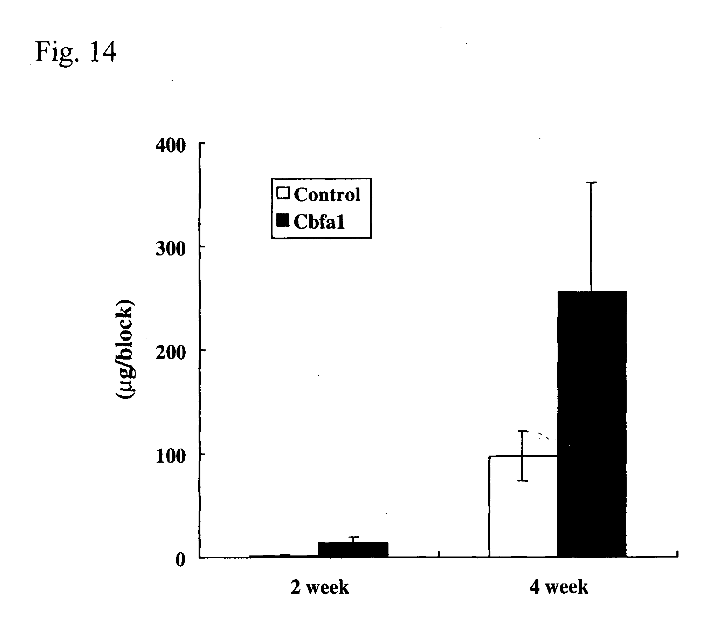

After the assay of alkaline phosphatase activity, the sediments of the blocks were disrupted by sonication in 20% formic acid, and incubated at 4°C for 4 days. After desalting with the use of a PD-10 column (Amersham Pharmacia Biotech), osteocalcin was extracted with 10% EDTA and 100 mM Tris (pH 7.4) and assayed the osteocalcin level using the Rat Osteocalcin EIA Kit (Biomedical Technologies Inc.) (Fig. 14).

### 2. Result of experimentation

### 1) Hematoxylin-eosin staining

According to the results of hematoxylin-eosin staining of the transplanted blocks (Figs. 11 and 12), the number of cells existing in pores of the blocks was larger and the size of ossification area was larger in the Cbfa1 (til-1) overexpressed cell.

### 2) Alkaline phosphatase activity

As is apparent from Fig. 13, the alkaline phosphatase activity of the cell in which Cbfa1 (til-1) overexpressed cell was higher than that of the control. Particularly, the enzyme activity 4 weeks after transplantation became as high as approximately 3 times that of the control.

### 3) Osteocalcin level

As is apparent from Fig. 14, the osteocalcin level in the Cbfa1 (til-1) overexpressed cell was higher than that in the control. Particularly, the osteocalcin level 4 weeks after transplantation became as high as approximately 2.5 times that of the control.

### Example 3: Experimentation for transplanting Cbfa1 gene-transfected osteoblasts to rat's back and bone defects

### 1. Experimental method

### 1) Transplantation into rat's back and into femur

The rat osteoblasts were infected with the Cbfa1 (til-1)-recombinant adenovirus vector at a moi of 500. Twenty four hours later, the cells were trypsinized and the cell suspension was soaked into the commercially available β-TCP block (average pore size: 200 µm in diameter, 5 mm x 5 mm x 5 mm (for subcutaneous transplantation), 2 mm x 2 mm x 2 mm (for bone defects transplantation), Olympus) at a cell density of 2,000,000 cells/ml. Next day, the blocks were transplanted to a rat's back and into the bone defects. Transplantation into the bone defects was conducted by producing holes (diameter: 2 mm, depth: 3 to 4 mm) in the frontal ends of left and right femora and by embedding the blocks in the holes (bone defects). As the control, blocks containing uninfected cells were similarly transplanted.

The subcutaneously transplanted block was excised 3 or 5 weeks after transplantation, the alkaline phosphatase activity and the osteocalcin level were assayed, and tissue sections were then observed. The block that had been transplanted into the femur was excised 3 or 8 weeks after transplantation, and tissue sections were then observed.

### 2. Result of experimentation

### 1) Hematoxylin-eosin staining

### Subcutaneous transplantation:

The number of cells existing in pores of the blocks with the Cbfa1 (til-1) overexpressed cell was larger, and bone formation was more advanced than that in the control, both 3 and 5 weeks after transplantation (Fig. 15A).

### Transplantation into bone defects:

3 weeks after transplantation, bone formation was advanced, and the dissected area of the femur was almost completely replaced with new bone tissue in the block with the Cbfa1 (til-1) overexpressed cell. In contrast, bone formation did not substantially occur in the case of the control. In the block with the Cbfa1 (til-1) overexpressed cell, bone formation was further advanced, and the bone defect was almost completely replaced with bone marrow tissues 8 weeks after transplantation. In contrast, the transplanted block still remained as it had been in the control (Fig. 15B).

### 2) Alkaline phosphatase activity and osteocalcin level

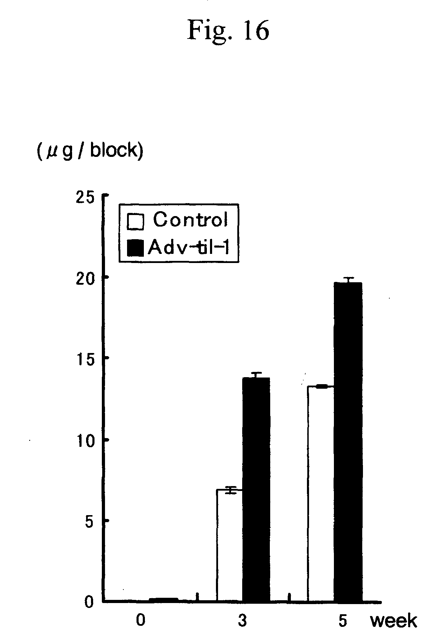

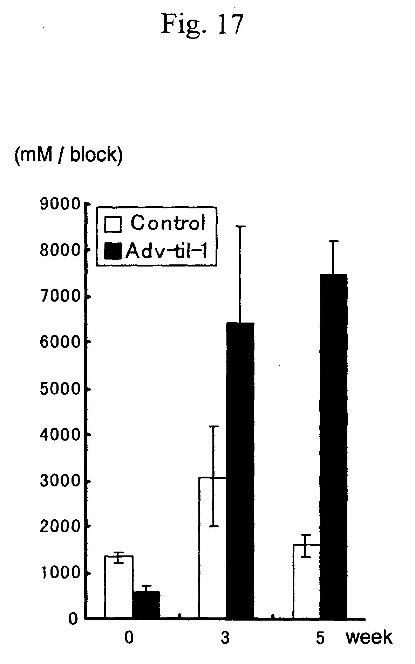

The alkaline phosphatase activity in the block with the Cbfa1 (til-1) overexpressed cell was approximately 2.0 times and approximately 4.7 times higher than that of the control 3 and 5 weeks after transplantation, respectively (Fig. 16). The osteocalcin level in the cell in which Cbfa1 (til-1) had been excessively expressed was approximately 2.0 times and approximately 1.5 times higher than that of the control 3 and 5 weeks after transplantation, respectively (Fig. 17).

All publications, patents, and patent applications cited herein are incorporated herein by reference in their entirety.

### Industrial Applicability

The present invention provides a method for effective *in vitro* tissue culture for bone/cartilage tissue regeneration using bone marrow cells. This method enables ultimate therapy for damaged tissues that cannot be regenerated *in vivo,* wherein a patient's own cells extracted from his/her body are cultured and organized *in vitro* to reconstruct tissues that are as similar as possible to those in the body, and the composite is returned to the body.

## Claims

1. A method for constructing bone/cartilage tissues, wherein an osteo-/chondro-inducible transcription factor gene is transfected into isolated cells, and the isolated cells are allowed to differentiate and proliferate.

2. The method according to claim 1, wherein the cells are derived from bone marrow.

3. The method according to claim 2, wherein the bone-marrow-derived cells are mesenchymal stem cells.

4. The method according to claim 2, wherein the bone-marrow-derived cells are osteoblasts.

5. The method according to any one of claims 1 to 4, wherein the cells are primary cells.

6. The method according to claim 5, wherein the cells are isolated from a patient.

7. The method according to any one of claims 1 to 6, wherein the osteo-/chondro-inducible transcription factor is at least one member selected from the group consisting of Cbfa1, Dlx-5, Bapx1, Msx2, Scleraxis, and Sox9.

8. The method according to any one of claims 1 to 7, wherein the osteo-/chondro-inducible transcription factor gene is transfected into the cells using an adenoviral or a retroviral vector.

9. The method according to any one of claims 1 to 8, wherein the cells transfected by the osteo-/chondro-inducible transcription factor genes proliferate in at least one scaffold selected from the group consisting of porous ceramics, collagen, polylactic acid, polyglycolic acid, and a complex thereof.

10. A method for constructing bone/cartilage tissues comprising the following procedures of:
1) inducing differentiation of bone-marrow-derived cells isolated from a body with the aid of at least one member selected from the group consisting of dexamethasone, immunosuppressive factors, bone morphogenetic proteins, and osteogenic cytokines;
2) transfection of the osteo-/chondro-inducible transcription factor gene into the cells using an adenoviral or retroviral vector; and
3) proliferation of the cells in at least one scaffold selected from the group consisting of porous ceramics, collagen, polylactic acid, polyglycolic acid, and a complex thereof.

11. Implants containing the bone/cartilage tissues constructed by the method according to any one of claims 1 to 10.

12. The implants according to claim 11, which further contain at least one scaffold selected from the group consisting of porous ceramics, collagen, polylactic acid, polyglycolic acid, and a complex thereof.

13. Implants containing cells, in which the osteo-/chondro-inducible transcription factor gene is transfected, and at least one member selected from the group consisting of porous ceramics, collagen, polylactic acid, polyglycolic acid, and a complex thereof.
